# EUROPEAN PATENT APPLICATION

(11) **EP 4 691 378 A1**
(43) Date of publication of application: **11.02.2026**
(21) Application number: 24932671.1
(22) Date of filing: 30.09.2024
(51) Int. Cl.: A61B 17/04

(54) **JOINT LIGAMENT ALL-SUTURE REPAIR ASSEMBLY**

(30) Priority: 29.03.2024 CN 202410371523
(71) Applicant: Star Sports Medicine Co., Ltd., 100176 Beijing (CN)
(72) Inventor: DONG, Wenxing, Beijing 100176 (CN); LI, Zhengrong, Beijing 100176 (CN); YIN, Jichen, Beijing 100176 (CN); GUO, Tingting, Beijing 100176 (CN)
(74) Representative: Angerhausen, Christoph
(86) International application number: PCT/CN2024/123010
(87) International publication number: WO 2025/200368

(57) **Abstract**

The present application relates to a full-suture repair assembly for joint ligaments, including a suture fixing loop plate and a suture fixing loop ring. The suture fixing loop plate is a flexible full-suture loop plate, is of a ring structure in an initial state, and closes and contracts radially under traction to form a flat disc structure with a closed inner ring. The suture fixing loop ring is formed by back-and-forth threading of a traction line on upper and lower surfaces of the suture fixing loop plate in a thickness direction, and includes tightening segments, a non-return structure, and self-locking knots.

## Description

### CROSS REFERENCE TO RELATED APPLICATION

This application claims priority to Chinese Patent Application No. 202410371523.0, filed on March 29, 2024 and entitled "FULL-SUTURE REPAIR ASSEMBLY FOR JOINT LIGAMENTS", which is hereby incorporated by reference in its entirety.

### TECHNICAL FIELD

The present application relates to the technical field of medical instruments, and specifically, to a full-suture repair assembly for joint ligaments.

### BACKGROUND

Currently, the reconstruction of injured ligaments mainly depends on suture anchors, full-suture anchors, interfacial screws, and looped titanium plates to fix ligament grafts. The suture anchors, the full-suture anchors, and the interfacial screws are used for intraosseous fixation, the suture anchors and the full-suture anchors used for the fixation of soft tissues and bones cannot repair or fix cruciate ligaments of knee joints, tibiae and fibulae, acromioclavicular joints, etc., while the interfacial screws need to be used with the looped titanium plates to reconstruct the cruciate ligaments. Moreover, preoperative drilling of large-diameter bone tunnels incurs significant bone damage, which makes the fixation prone to loosening and ultimately leads to fixation failure.

The looped titanium plates are used for extraosseous suspension fixation, and are mainly made of metallic hard materials. When in contact with a hard bone surface, the looped titanium plate poses a risk of collapsing the bone cortex to cause compressive fractures. In addition, the friction and impact between hard objects may lead to the "windshield wiper effect" and "bungee effect", resulting in secondary damage to the bone. From a surgical perspective, a large-diameter bone tunnel needs to be drilled before surgery, and a metallic graft needs to be inserted through the bone tunnel during surgery. Prolonged friction of flexible sutures with the metallic graft after surgery may lead to suture breakage, increasing the risk of surgery.

Currently, flexible materials have been used instead of metallic materials to solve the problem of secondary damage of sutures and looped plates to bones and surgical risks. For example, CN219250292U discloses a loop ring structure used in ligament or tendon injury repair surgery, but this loop ring structure ultimately forms three-dimensional balls and forms protrusions at repair sites to affect the repair effect. CN117017569A discloses a full-suture polymer button for suspension plasty of carpal and palmar arthritis, which does not form a three-dimensional structure, but is only suitable for small joints, has low fixation strength, and cannot be used for other joints.

Therefore, it is necessary to propose a full-suture repair assembly for joint ligaments to effectively solve the above problems in clinical use.

### SUMMARY

One of the objectives of the present application is to provide a full-suture repair assembly for joint ligaments, which can not only avoid damage of a rigid loop plate to bones, but also ensure fixation strength and stability, with good repair effect and strong clinical applicability.

In order to achieve the above objective, the present application provides a full-suture repair assembly for joint ligaments, including a suture fixing loop plate and a suture fixing loop ring,

the suture fixing loop plate is a flexible full-suture loop plate, is of a ring structure in an initial state, and closes and contracts radially under traction to form a flat disc structure with a closed inner ring; the suture fixing loop ring is formed by back-and-forth threading of a traction line on upper and lower surfaces of the suture fixing loop plate in a thickness direction;

the traction line is threaded on the upper surface of the suture fixing loop plate in the thickness direction to form a plurality of tightening segments, the traction line is threaded on the lower surface of the suture fixing loop plate in the thickness direction to form a plurality of coils, one of the coils extends downward to form an initial coil, and free ends of the traction line are interwoven on the initial coil to form a non-return structure of the suture fixing loop ring; and
after the non-return structure is formed, the two free ends of the traction line are threaded back to the upper surface of the suture fixing loop plate in the thickness direction of the suture fixing loop plate, and pass through the tightening segments to form self-locking knots of the suture fixing loop ring.

According to an embodiment in the first aspect of the present application, the suture fixing loop plate is formed by interweaving of a hollow tubular suture, the hollow tubular suture has a diameter of 1 to 3 mm, and its two ends wrap up half of the circumference.

According to any of the foregoing embodiments in the first aspect of the present application, a first free end of the hollow tubular suture is threaded into a chamber of the hollow tubular suture by a length and then threaded out in the entry direction;
a second free end of the hollow tubular suture is threaded into the chamber at the entrance of the hollow tubular suture from the first free end, threaded in a direction opposite to the first free end, and threaded out at the exit of the hollow tubular suture from the first free end; and
exit portions of the hollow tubular suture are cut off at exit roots of the two free ends to form the suture fixing loop plate in the initial state.

According to any of the foregoing embodiments in the first aspect of the present application, in the initial state, the suture fixing loop plate is a full-suture flexible ring, with an outer ring diameter of 6 to 16 mm and an inner ring diameter of 3 to 12 mm.

According to any of the foregoing embodiments in the first aspect of the present application, the tightening segments are equidistantly distributed on the upper surface of the suture fixing loop plate in the thickness direction, and the length of each tightening segment is 2 to 6.5 mm.

According to any of the foregoing embodiments in the first aspect of the present application, the tightening segments include four segments.

According to any of the foregoing embodiments in the first aspect of the present application, the two free ends of the traction line and the initial coil are arranged on the same side of the suture fixing loop plate, where one of the free ends is threaded into the segment of the initial coil near the free end, and then threaded through a length in a direction away from the free end before exiting the initial coil; and
the other free end is threaded into the initial coil from the exit portion of the previous free end at the initial coil, threaded through a length in a direction opposite to the exposed position of the previous free end, and then threaded out the initial coil from the entry position of the previous free end at the initial coil, and the threading position of the traction line in the initial coil forms the non-return structure.

According to any of the foregoing embodiments in the first aspect of the present application, the two free ends of the traction line extend towards the suture fixing loop plate and pass through the overall thickness direction of the suture fixing loop plate after extending out of the non-return structure, and the free ends are arranged radially outside the tightening segments at the threading position of the suture fixing loop plate.

According to any of the foregoing embodiments in the first aspect of the present application, after passing through the suture fixing loop plate, the two free ends of the traction line extend over two tightening segments arranged oppositely on the upper surface of the suture fixing loop plate in the thickness direction, and are wound back from the outer ring to the inner ring of the suture fixing loop plate.

According to any of the foregoing embodiments in the first aspect of the present application, the traction line is of a single suture structure; and the tightening segments, the non-return structure, and the self-locking knots are all formed by threading and winding the single traction line on the suture fixing loop plate.

By utilizing the flexibility and initial ring structure of the suture fixing loop plate, the suture fixing loop plate can deform and pass through small bone tunnels, which not only reduces wear on the bone tunnels but also reduces bone loss; during surgery, the flexible loop plate made by threading of the hollow tubular suture easily tightens into knots, thereby effectively enhancing the fixation strength and stability of the repair assembly; by combining the tightening effect of the suture fixing loop ring on the suture fixing loop plate, the suture fixing loop plate with the ring structure is radially closed and contracted, and ultimately transforms into a closed flat disc structure after surgery.

The radial tightening deformation of the suture fixing loop can effectively avoid bulging into a ball. After tightening, the suture fixing loop plate can maintain a good fit with the bone surface, thereby reducing the irritation of nodules to soft tissues, reducing the occurrence of inflammation, and bringing better treatment experience to patients.

The traction line for the suture fixing loop ring is threaded back and forth on the upper and lower surfaces of the suture fixing loop plate in the thickness direction to form the plurality of tightening segments, which can provide a basis for the non-return structure and provide uniform extrusion force for the subsequent tightening deformation of the suture fixing loop plate, thereby controlling the creep path of the initial suture fixing loop plate and achieving the technical purpose of closing and tightening the suture fixing loop plate radially without bulging in the thickness direction.

The traction line is threaded on the lower surface of the suture fixing loop plate in the thickness direction to form the plurality of coils, which provides a basis for the formation of the suture fixing loop ring. Meanwhile, one of the coils extends downwards to form the initial coil. Combined with the interweaving of the free ends of the traction line on the initial coil, the non-return structure of the suture fixing loop ring is formed at the bottom of the initial coil. The two free ends of the traction line are mutually threaded through the structure of the suture fixing loop ring to increase the contact area between the suture coils, and the threading overlap part increases local friction under force to effectively hinder the extension of the coils.

After the non-return structure is formed, the two free ends of the traction line are threaded back to the upper surface of the suture fixing loop plate in the thickness direction of the suture fixing loop plate, and wound back to pass through the tightening segments to form self-locking knots on the suture fixing loop plate for extrusion locking, and the locking force increases with the increase of force on the coils to ultimately form the non-return structure, thereby preventing the suture fixing loop ring from loosening during fixation and achieving a good repair effect.

The full-suture repair assembly for joint ligaments in the present application is suitable for the repair, fixation, and reconstruction of tendons and ligaments in shoulder, knee, elbow, finger, ankle, wrist, and foot joints, applies to multiple sites, and has extremely high clinical practicality.

Other features and advantages of the present disclosure will be described in detail in the following specific embodiments.

### BRIEF DESCRIPTION OF THE DRAWINGS

In order to explain the technical solutions in the embodiments of the present application more clearly, accompanying drawings required to use in the embodiments will be simply introduced below. It should be understood that the following drawings show merely some embodiments of the present application and should not be regarded as limiting the scope, and other relevant drawings may be obtained based on these drawings by those of ordinary skill in the art without any creative efforts.
FIG. 1 is a schematic diagram of an overall structure of a full-suture repair assembly for joint ligaments in the present application;
FIG. 2 is a schematic diagram of a side structure of FIG. 1;
FIG. 3 is a schematic diagram of a side structure of FIG. 1 from another perspective;
FIG. 4 is a schematic structural diagram of a suture fixing loop plate in an initial state;
FIG. 5 is a schematic structural diagram of a process of threading formation of a suture fixing loop plate;
FIG. 6 is a schematic structural diagram of a process of threading formation of tightening segments by a suture fixing loop ring on a suture fixing loop plate;
FIG. 7 is a schematic diagram of a distribution structure of tightening segments and a non-return structure on the suture fixing loop plate;
FIG. 8 is a schematic structural diagram of a process of threading formation of a non-return structure;
FIG. 9 is a schematic structural diagram of a process of threading formation of self-locking knots;
FIG. 10 is a schematic diagram of an overall structure of a suture fixing loop ring; and
FIG. 11 is a schematic diagram of a usage process of the full-suture repair assembly for joint ligaments in a surgical process.

Reference numerals:
1 - suture fixing loop plate; 1a - hollow tubular suture;
2 - suture fixing loop ring; 2a - tightening segment; 2b - non-return structure; 2c - initial coil; 2d - self-locking knot.

### DETAILED DESCRIPTION

In order to make the objectives, technical solutions, and advantages of the embodiments of the present application clearer, the technical solutions in the embodiments of the present application will be clearly and completely described below in conjunction with the accompanying drawings therein. Apparently, the described embodiments are some of the embodiments of the present application, not all of them. Generally, components in the embodiments of the present application, described and shown in the drawings, may be arranged and designed with various different configurations.

In the description of the present application, it should be noted that the orientation or positional relationships indicated by the terms "inner", "outer", etc. are based on the orientation or positional relationships shown in the drawings, or the orientations or positional relationships commonly placed when the product of the present application is used. The orientation or positional relationships are merely for the convenience of describing the present application and simplifying the description, do not indicate or imply that a device or component referred to has a specific orientation or is constructed and operated in a specific orientation, and therefore, cannot be understood as limitations of the present application. In addition, the terms "first", "second", etc. are merely used to distinguish descriptions and cannot be understood as indicating or implying relative importance.

In the description of the present application, it should also be noted that, unless otherwise specified and limited, the terms "arranged" and "connected" should be understood in a broad sense. For example, the "connected" may be fixedly connected, detachably connected, integrally connected, directly connected, indirectly connected by a medium, or connected internally between two components. A person of ordinary skill in the art may understand the specific meanings of the above terms in the present application according to specific circumstances.

A full-suture repair assembly for joint ligaments in the present application is mainly used in the process of ligament injury repair and reconstruction. Specifically, by using the flexibility of a full-suture fixing loop plate, the structure of the loop plate and line segments combined on the loop plate are changed to improve the shape of the loop plate during repair surgery, so that the loop plate is radially tightened and locked under the pulling and retracting action of a tension line.

Compared with existing fixing loop plate structures, the radial contraction closure deformation can avoid the final formation of three-dimensional balls, reduce the thickness of the fixing loop plate, and avoid irritation to soft tissues, thereby improving the repair effect of joint ligaments, reducing the occurrence of inflammation, and avoiding patient's discomfort caused by enlarged nodules.

Meanwhile, the flexible ring structure transforms into a flexible disc structure after deformation, which can maintain a flat structure in an initial state, maintain good fit with a bone surface, increase fixation strength, reduce surgical risks, and provide patients with good treatment experience.

After radial contraction closure, the suture fixing loop plate reduces fiber gaps and increases overall stiffness. The suture fixing loop plate does not undergo deformation any more, but is firmly suspended on a bone opening to enhance fixation stability.

A traction line threaded through the suture fixing loop plate provides a basis for forming a suture fixing loop ring that fixes a ligament, and the threading path of the traction line on the suture fixing loop plate can be utilized to control the deformation of the fixing loop plate. Moreover, the threading of loop coils can be utilized to increase the contact area between sutures and enhance friction, thereby forming a non-return structure and a self-locking structure of the suture fixing loop ring without additional components, and effectively ensuring the fixation strength and stability of the full-suture repair assembly.

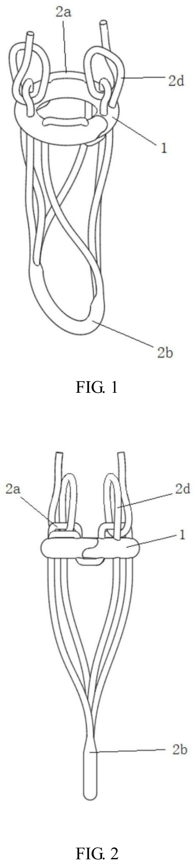

With reference to FIG. 1, combined with FIGS. 2, 3, and 10, reference numerals therein represent specific structures. A full-suture repair assembly for joint ligaments in the present application mainly includes a suture fixing loop plate 1 and a suture fixing loop ring, the suture fixing loop ring is formed by back and forth threading of a traction line in a thickness direction of the suture fixing loop plate 1.

The suture fixing loop plate 1 is a flexible loop plate, with a flexible ring structure in an initial state. Specifically, the suture fixing loop plate 1 is a flexible ring before deformation. In the surgical process, the flexible ring may undergo radial closure contraction deformation under the traction of a traction line and then transform into a flexible disc structure.

The suture fixing loop plate 1 is of a flat structure both before and after surgery. Specifically, the suture fixing loop plate 1 is formed by interweaving an ultra-high molecular weight polyethylene hollow tubular suture 1a. By using the flexibility and variability of the suture, the flexible ring in the initial state before surgery is enabled to pass through small bone tunnels, which not only incurs little damage to the bone tunnels but also reduces the amount of bone loss.

During surgery, the flexible ring loop plate formed by interweaving easily deforms and transforms into a fixing disc loop plate structure. After surgery, the suture fixing loop plate 1 remains flat, achieving the technical purposes of avoiding bulging into balls, reducing the irritation of nodules to soft tissues, and reducing the risk of inflammation caused by friction, and bringing better treatment experience to patients.

The traction line mainly functions to form a fixing loop ring that may be connected to a graft, transmit tension to drive the radial closure contraction of the suture fixing loop plate, and prevent the extension of coils that make up the fixing loop ring.

Specifically, the traction line is threaded back and forth on upper and lower surfaces of the suture fixing loop plate 1 in a thickness direction, to form the suture fixing loop ring bonded to the suture fixing loop plate 1.

Further, the traction line is threaded on the upper surface of the suture fixing loop plate in the thickness direction to form a plurality of tightening segments 2a that drive the deformation of the flexible ring loop plate. Through such arrangement, the tension can be effectively transmitted to the ring loop plate to generate uniform compression, enabling the flexible ring loop plate 1 to contract towards its center to form a disc structure.

The traction line is threaded on the lower surface of the suture fixing loop plate in the thickness direction to form a plurality of coils, one of the coils extends downward to form an initial coil 2c, and free ends of the traction line are interwoven on the initial coil 2c to form a non-return structure 2b of the suture fixing loop ring.

Specifically, two free ends of the traction line are sequentially interwoven at a bottom of the initial coil to form the non-return structure 2b of the suture fixing loop ring 2. A ligament graft or the like generates a relative opposite force in the fixation process. The non-return structure 2b is mainly used to prevent the opposite force from acting on the suture fixing loop ring 2, which can effectively hinder the extension of the coils. The two free ends of the traction line are interwoven on the initial coil 2c to increase the contact area between the suture coils, and their overlap part increases local friction under force to effectively hinder the extension of the coils.

The non-return structure 2b obtained by interweaving does not require additional settings of other components. On the premise of simplifying the composition of the non-return structure 2b, the non-return effect can be effectively ensured by increasing the local contact friction.

After the non-return structure 2b of the suture fixing loop ring 2 is formed, the two free ends of the traction line are threaded back to the upper surface of the suture fixing loop plate 1 in the thickness direction of the suture fixing loop plate 1, and wound back to pass through the tightening segments 2a to form self-locking knots 2d of the traction line for extrusion locking, and the locking force increases with the increase of force on the coils to ultimately form the non-return structure.

The full-suture repair assembly for joint ligaments in the present application can effectively apply to the repair, fixation, and reconstruction of tendons and ligaments in the shoulder, knee, elbow, finger, ankle, wrist, and foot joints, with good clinical practicality.

In one specific embodiment, the suture fixing loop plate 1 is formed by interweaving a hollow tubular suture 1a made of an ultra-high molecular weight polyethylene material, the ultra-high molecular weight polyethylene material does not stimulate surrounding tissues and has stable chemical properties, making it more suitable for use in reduction therapy surgery.

Specifically, the hollow tubular suture 1a has a diameter of 1 to 3 mm, and its two ends wrap up half of the circumference.

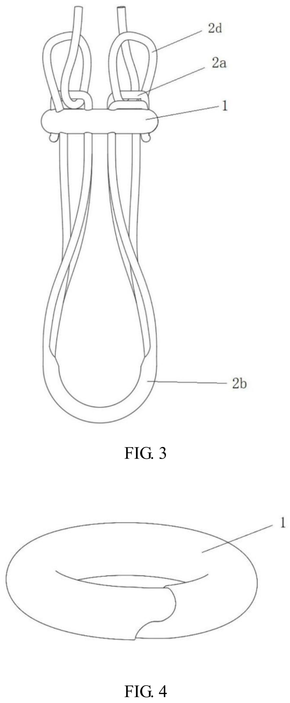

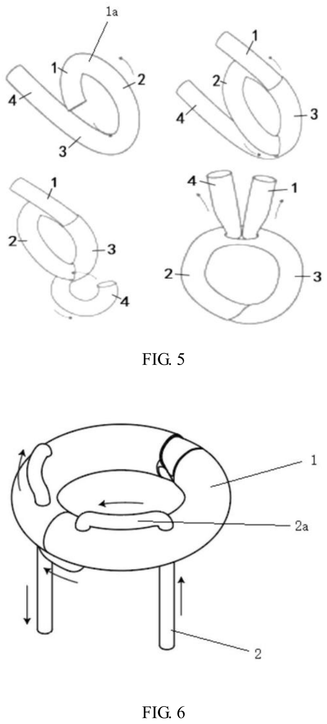

Referring to FIG. 4 which shows a structure of the suture fixing loop plate 1, and in combing with FIG. 5, in order to better illustrate the preparation process, 1a in FIG. 5 represents a structure of the hollow tubular suture 1a, while other numerals represent different segments of the hollow tubular suture 1a. When the suture fixing loop plate 1 in the initial state is prepared, a segment of the hollow tubular suture 1a is interwoven. In the specific interweaving process, the first free end of the hollow tubular suture 1a is wound back into the tubular segment of the hollow tubular suture 1a, and the hollow tubular suture 1a is threaded out in the threading direction after passing through a length.

The second free end of the hollow tubular suture 1a is threaded into the tubular segment of the hollow tubular suture 1a at the entrance of the hollow tubular suture 1a from the first free end, and the hollow tubular suture 1a is threaded in a direction opposite to the first free end of the hollow tubular suture 1a. The hollow tubular suture 1a extends out at the exit of the hollow tubular suture 1a from the first free end after passing through a length. Therefore, the interweaving of the two free ends in the hollow tubular suture 1a is completed.

After the interweaving is completed, exit portions of the two free ends of the hollow tubular suture 1a are cut off at exit roots of the two free ends to form the suture fixing loop plate 1 in the initial state.

The suture fixing loop plate obtained in this way is specifically in the form of a flexible loop ring, and can pass through bone tunnels before surgery based on its flexibility before deformation. Meanwhile, the suture fixing loop plate 1 can undergo peristaltic deformation under the action of the traction line, and close and tighten radially to transform inner ring closure into a disc structure.

In the initial state, the suture fixing loop plate 1 is a full-suture flexible ring, with an outer ring diameter of 6 to 16 mm and an inner ring diameter of 3 to 12 mm.

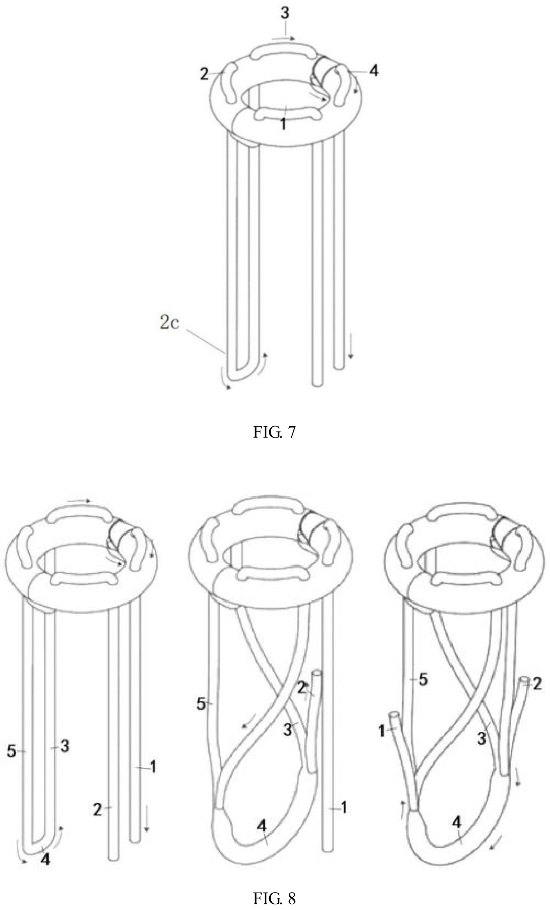

Refer to FIGS. 6 and 7, numerals in FIG. 6 represent specific structures. In order to better illustrate the threading process, 1 to 4 in FIG. 7 represent different segments. From the perspective of controlling the deformation of the suture fixing loop plate 1 during surgery, the tightening segments 2a are equidistantly distributed on the upper surface of the suture fixing loop plate 1, and the length of each tightening segment 2a on the suture fixing loop plate 1 is 2 to 6.5 mm. The traction line is also made of ultra-high molecular weight polyethylene, and is specifically a non-absorbent surgical suture made of an ultra-high molecular weight polyethylene material.

Further, based on the structure of the flexible loop ring, the tightening segments 2a include four segments, the non-return structure 2b is arranged between the second tightening segment and the third tightening segment, and the non-return structure 2b is arranged below the lower surface of the suture fixing loop plate 1.

In the specific threading process in FIG. 6, the traction line is threaded from the lower flat surface of the suture fixing loop plate 1, and equidistantly threaded counterclockwise back and forth on the upper and lower surfaces of the suture fixing loop plate 1 along the circumference. After the second segment is threaded, a length is reserved, and then the third segment is threaded. Thus, the initial coil 2c with a non-return function in FIG. 7 is formed between the second segment and the third segment.

After four turns of threading, a fixing loop ring with the initial coil 2c is formed. Then, the two free ends of the traction line are sequentially threaded through the bottom of the initial coil 2c, with a length of 10 to 30 mm, to form the non-return structure of the suture fixing loop ring.

Specifically, the two free ends of the traction line and the initial coil 2c are arranged on the same side of the suture fixing loop plate. As shown in the figures, the two free ends and the initial coil 2c are arranged on the lower side of the suture fixing loop plate.

The two free ends of the traction line are sequentially threaded in the initial coil 2c. Specifically, one of the free ends of the traction line is threaded into the segment of the initial coil 2c near the free end, and then threaded through a length in a direction away from the free end before exiting the initial coil 2c.

As shown in FIG. 8, the free end 1 is a threading tail end described below, and the free end 2 is a threading head end described below. The threading head end and the threading tail end are defined relative to the tightening segments and the threading direction of the initial coil 2c on the traction line for ease of explanation.

The threading tail end of the traction line is first threaded. The initial coil 2c is specifically of a closed U-shaped structure, including two vertical segments. The threading tail end of the traction line is threaded into the initial coil 2c from the vertical segment near the threading tail end, passes through the bottom of the initial coil 2c, and exits from the vertical segment on the other side of the initial coil 2c.

The other free end is threaded into the initial coil 2c from the exit portion of the previous free end at the initial coil 2c, threaded through a length in a direction opposite to the exposed position of the previous free end, and then threaded out the initial coil 2c from the entry position of the previous free end at the initial coil 2c.

With reference to the structure in the figure, after the threading of the threading tail end on the initial coil 2c is completed, the threading of the threading head end on the initial coil 2c is carried out. The threading head end is threaded into the initial coil 2c at the extension position of the initial coil 2c, then passes through the initial coil 2c by a length in a direction opposite to the exposed position of the threading tail end, and exits the initial coil 2c from the entry position of the threading tail end at the initial coil 2c.

Through the above threading process, a non-return structure that is threaded through the structure of the suture fixing loop ring may be obtained to increase the contact area between coil sutures, and the overlap part increases local friction under force to effectively hinder the extension of the coils for the suture fixing loop ring.

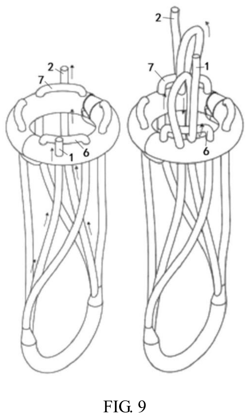

With reference to FIGS. 8 and 9, in order to better illustrate the threading process, numerals 1 to 5 in FIG. 8 and numerals 1, 2, 6, and 7 in FIG. 9 represent different segments to facilitate understanding of the threading steps. The two free ends of the traction line extend towards the suture fixing loop plate 1 and pass through the overall thickness direction of the suture fixing loop plate 1 after extending out of the initial coil 2c of the non-return structure 2b. The free ends are arranged radially outside the tightening segments 2a at the threading position of the suture fixing loop plate 1.

Further, after passing through the suture fixing loop plate 1, the two free ends of the traction line extend over two tightening segments 2a arranged oppositely on the upper surface of the suture fixing loop plate 1 in the thickness direction, respectively.

Preferably, the two free ends of the traction line are threaded through two diagonal tightening segments 2a on the upper surface of the suture fixing loop plate 1 in the thickness direction, and pass through the radial outer sides of the tightening segments 2a relative to the suture fixing loop plate 1.

After passing through the suture fixing loop plate 1, the two free ends extend upwards and are wound back from the outer ring to the inner ring of the suture fixing loop plate 1. In the back winding process, the two free ends pass through the gaps between the tightening segments 2a and the suture fixing loop plate 1 to ultimately form self-locking knots 2d.

After the threading of the non-return structure is completed, the two free ends of the traction line are threaded through the suture fixing loop plate 1 again, and wound around the two diagonal tightening segments 2a on the upper surface of the suture fixing loop plate 1 to achieve extrusion locking. The locking force increases with the increase of force on the non-return structure 2b, and a self-locking structure with a non-return function is ultimately formed.

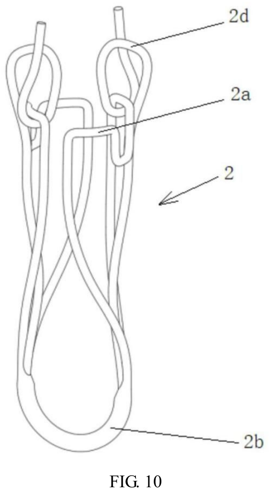

With reference to FIG. 10, the traction line in the present application is of a single structure. The tightening segments 2a, the non-return structure 2b, the initial coil 2c, and the self-locking knots 2d are all formed by threading and winding the single traction line on the suture fixing loop plate 1. By combining the closed locking of the flexible loop ring under the action of the traction line and the flexible disc structure transformed from the flexible ring structure with unchanged shape and increased fixation strength after deformation, the overall fixation strength and stability of the repair assembly can be increased.

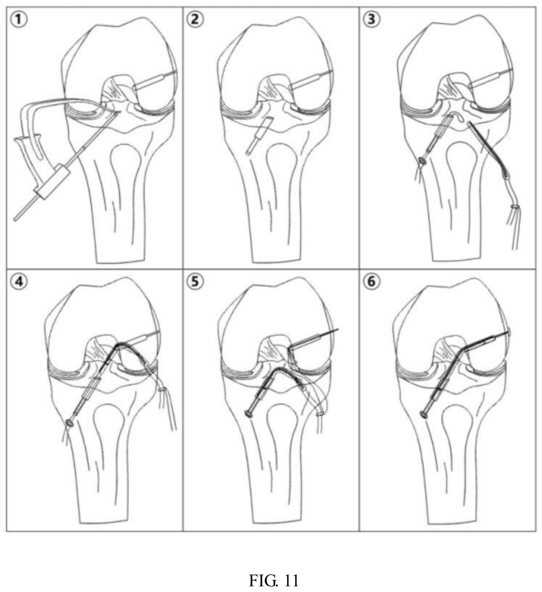

With reference to FIG. 11, the surgical steps are described in order of numerals as follows.
1. Bone tunnel positions are found with a guider and a guide pin and drilled with a drill bit of a suitable model through the guider; 2. Two bone tunnels are obtained, and a joint ligament is fixed by two full-suture repair assemblies for joint ligaments; 3. The suture fixing loop ring of one full-suture repair assembly is threaded through the bone tunnels by using a thread guide and connected to a graft, and the suture fixing loop plate is located on the outer bone cortex of the bone tunnels; 4. The suture fixing loop ring is tightened through the traction line, and the graft enters the drilled hole and is knotted for fixation; 5. The suture fixing loop ring of the other full-suture repair assembly is threaded through the bone tunnels by using the thread guide, and the graft is pre-connected to the other repair assembly; 6. The traction line is pulled to allow the suture fixing loop plate to pass through the hole to the outer bone cortex, the coils of the suture fixing loop ring are tightened through the traction line, the graft enters the drilled hole, and the status of the graft is adjusted before fixation.

The main working principle of this scheme is as follows. When the distal end of the traction line is subjected to a pulling force, the force is transmitted along the suture to the suture fixing loop plate 1 to extrude the fixing plate uniformly, the suture fixing loop plate 1 contracts towards its center to form disc knots, which are then suspended on the bone cortex. After the flexible suture fixing loop plate 1 contracts, the fiber gap decreases, the overall stiffness increases, and the suture fixing loop plate 1 does not deform any more but is stably suspended at the openings of the bone tunnels to provide effective fixation.

When the suture of the suture fixing loop plate is subjected to the pulling force, the suture undergoes radial contraction from coarse to fine, and the overlap part at the bottom of the non-return structure 2b is extruded to increase friction. Meanwhile, the upper loop of the suture fixing loop plate 1 exerts downward pressure on the free ends of the suture to hinder the movement and retraction of the free ends, thereby preventing the non-return structure 2b from elongating under tensile load.

The suture fixing loop plate 1 in the present application may be disc-shaped, rectangular, triangular, or polygonal in addition to ring-shaped.

The traction line is threaded back and forth through the suture fixing loop plate 1, or the traction line cannot be adjusted. In addition to the single traction line, two, three, or four traction lines may simultaneously threaded to weave the non-return structure 2b.

In addition to the use of one suture fixing loop plate 1, two suture fixing loop plates 1 may be axially overlapped, and the traction line is simultaneously threaded on the two suture fixing loop plates 1 to increase the fixation strength.

The turns of threading and weaving of the traction line on the suture fixing loop plate 1 may be adjusted according to the practice to increase the reliability of applying the pulling force to the suture fixing loop plate 1, thereby increasing the force on the suture fixing loop plate 1.

In addition, the traction line may be threaded into the suture fixing loop plate 1 in a cross, side-by-side or other manner to form knots on the suture fixing loop plate 1 according to the specified path, which will not be elaborated here.

The full-suture repair assembly for joint ligaments in the present application is suitable for the repair and reconstruction of tendons and ligaments in the shoulder, knee, elbow, finger, ankle, wrist, and foot joints. Among these applicable sites, the knee joint, as the joint area with the highest load in human activities, requires the highest fixation strength of the product. Therefore, the full-suture repair assembly for joint ligaments is accepted based on the clinical requirements of the knee joint.

The static fixation strength of the full-suture repair assembly for joint ligaments in the present application was compared with that of an existing looped titanium plate under the same experimental conditions. Test records are shown in the following table:

Experimental record table of fixation strength test

| **Name** | **Serial number** | **Coil elongation/mm** | **Load/N** | **Failure mode** |
|---|---|---|---|---|
| Full suture repair assembly for joint ligaments in the present application | 1 | 2.2 | 1047.49 | Cortical rupture |
| | 2 | 2.8 | 1222.58 | |
| | 3 | 1.1 | 1035.63 | |
| | 4 | 1.4 | 1380.56 | |
| | 5 | 1.2 | 1259.49 | |
| | Mean ± SD | 1.74±0.66 | 1189.15±131.4 | |
| Looped titanium plate | 1 | 4.35 | 1176.45 | Cortical rupture |
| | 2 | 4.36 | 1321.02 | |
| | 3 | 4.2 | 1289.20 | |
| | 4 | 4.27 | 1184.36 | |
| | 5 | 4.18 | 1237.68 | |
| | Mean ± SD | 4.27±0.07 | 1241.74±56.76 | |

The literature Experimental Research on the Mechanical Properties of Looped Titanium Plates for the Reconstruction of Anterior Cruciate Ligaments specifies: a. The maximum mean ± SD load of the cruciate ligament of the knee joint is 380.35±76.45 N, that is, the fixation strength of the graft on the ligament is more than 500 N to meet clinical usage requirements; b. When the deformation of the fixing object (coil elongation) is more than 3 mm, fixation failure is determined, that is, the coil elongation of the fixing object in fatigue testing shall not be more than 3 mm.

According to the experimental record table of fixation strength test under static conditions, it can be seen that the mean fixation strength of the flexible suture fixing loop plate with the full-suture in the present application was 1189.15 N, which was close to the mean fixation strength 1241 N of the metallic looped titanium plate; as a flexible material, the fixation strength of the flexible suture fixing loop plate was close to that of metal materials and far greater than 500 N as clinically required; the mean elongation of the coil of the full-suture fixing loop ring, i.e., the non-return structure in the present application, was 1.74 mm, which was less than the mean elongation 4.27 mm of the coil of the metallic looped titanium plate; and even in static fixation strength test, the stability of the coil of the suture fixing loop ring still met the clinical usage requirements.

It should be noted that the features in the embodiments of the present application may be combined with each other on a non-conflict basis.

Described above are merely preferred embodiments of the present application, and the present application is not limited thereto. For a person skilled in the art, the present application may have various modifications and variations. Any modifications, equivalent substitutions, improvements, and the like made within the spirit and principle of the present application shall fall within the protection scope of the present application.

## Claims

1. A full-suture repair assembly for joint ligaments, comprising a suture fixing loop plate and a suture fixing loop ring, wherein
the suture fixing loop plate is a flexible full-suture loop plate, is of a ring structure in an initial state, and closes and contracts radially under traction to form a flat disc structure with a closed inner ring, the suture fixing loop plate is formed by interweaving of a hollow tubular suture, and the suture fixing loop ring is formed by back-and-forth threading of a traction line on upper and lower surfaces of the suture fixing loop plate in a thickness direction;
the traction line is threaded on the upper surface of the suture fixing loop plate in the thickness direction to form a plurality of tightening segments, the traction line is threaded on the lower surface of the suture fixing loop plate in the thickness direction to form a plurality of coils, one of the coils extends downward to form an initial coil, and free ends of the traction line are interwoven on the initial coil to form a non-return structure of the suture fixing loop ring; and
after the non-return structure is formed, two free ends of the traction line are threaded back to the upper surface of the suture fixing loop plate in the thickness direction of the suture fixing loop plate, and pass through the tightening segments to form self-locking knots of the suture fixing loop ring.

2. The full-suture repair assembly for joint ligaments according to claim 1, wherein the hollow tubular suture has a diameter of 1 to 3 mm, and its two ends wrap up half of the circumference.

3. The full-suture repair assembly for joint ligaments according to claim 2, wherein a first free end of the hollow tubular suture is threaded into a chamber of the hollow tubular suture by a length and then threaded out in the entry direction;
a second free end of the hollow tubular suture is threaded into the chamber at the entrance of the hollow tubular suture from the first free end, threaded in a direction opposite to the first free end, and threaded out at the exit of the hollow tubular suture from the first free end; and
exit portions of the hollow tubular suture are cut off at exit roots of the two free ends to form the suture fixing loop plate in the initial state.

4. The full-suture repair assembly for joint ligaments according to claim 3, wherein in the initial state, the suture fixing loop plate is a full-suture flexible ring, with an outer ring diameter of 6 to 16 mm and an inner ring diameter of 3 to 12 mm.

5. The full-suture repair assembly for joint ligaments according to claim 1, wherein the tightening segments are equidistantly distributed on the upper surface of the suture fixing loop plate in the thickness direction, and a length of each of the tightening segments is 2 to 6.5 mm.

6. The full-suture repair assembly for joint ligaments according to claim 5, wherein the tightening segments comprise four segments.

7. The full-suture repair assembly for joint ligaments according to claim 1, wherein the initial coil and the two free ends of the traction line are arranged on a same side of the suture fixing loop plate, one of the two free ends is threaded into a segment of the initial coil near the free end, and then threaded through a length in a direction away from the one of the free end before exiting the initial coil; and
the other one of the two free end is threaded into the initial coil from the exit portion of the previous free end at the initial coil, threaded through a length in a direction opposite to the exposed position of the previous free end, and then threaded out the initial coil from the entry position of the previous free end at the initial coil, and the threading position of the traction line in the initial coil forms the non-return structure.

8. The full-suture repair assembly for joint ligaments according to claim 1, wherein the two free ends of the traction line extend towards the suture fixing loop plate and pass through the overall thickness direction of the suture fixing loop plate after extending out of the non-return structure, and the two free ends are arranged radially outside the tightening segments at the threading position of the suture fixing loop plate.

9. The full-suture repair assembly for joint ligaments according to claim 1, wherein after passing through the suture fixing loop plate, the two free ends of the traction line extend over two tightening segments arranged oppositely on the upper surface of the suture fixing loop plate in the thickness direction, and are wound back from the outer ring to the inner ring of the suture fixing loop plate.

10. The full-suture repair assembly for joint ligaments according to claim 7, wherein the traction line is of a single suture structure; and the tightening segments, the non-return structure, and the self-locking knots are all formed by threading and winding the single traction line on the suture fixing loop plate.
